# EUROPEAN PATENT APPLICATION

(11) **EP 4 190 403 A1**
(43) Date of publication of application: **07.06.2023**
(21) Application number: 21850933.9
(22) Date of filing: 28.07.2021
(51) Int. Cl.: A61Q 5/06, A61K 8/34, A61K 8/69, A61K 8/81

(54) **HAIRSPRAY COMPOSITION AND HAIRSPRAY PRODUCT**

(30) Priority: 28.07.2020 JP 2020127574
(71) Applicant: Daizo Corporation, Osaka-shi, Osaka 552-0013 (JP)
(72) Inventor: YOKOGI, Ayako, Kyoto-shi, Kyoto 613-0916 (JP); MONJYU, Ryosuke, Sashima-gun, Ibaraki 306-0314 (JP)
(74) Representative: Bryn Aarflot AS
(86) International application number: PCT/JP2021/027917
(87) International publication number: WO 2022/025120

(57) **Abstract**

Provided is a spray composition for hair styling comprising a styling resin and a solvent, wherein the solvent comprises hydrofluoroolefin having a boiling point of 5 to 40°C and an alcohol.

## Description

### TECHNICAL FIELD

The present invention relates to a spray composition for hair styling and a spray product for hair styling. More specifically, the present invention relates to a spray composition for hair styling and a spray product for hair styling both having an excellent drying property and an excellent setting power.

### BACKGROUND ART

Conventionally, aerosol products for styling have been developed. Patent Document 1 discloses an aerosol composition for hair styling, comprising a styling resin, an alcohol, and a propellant (hydrofluoroolefin having a boiling point of less than 5°C).

### PRIOR ART DOCUMENT

### Patent Document

Patent Document 1: US 2013/0280178 A

### SUMMARY OF THE INVENTION

The invention described in Patent Document 1 uses hydrofluoroolefin having a boiling point of -19°C and a low combustibility, considering fire safety. However, since the invention described in Patent Document 1 is a propellant having a boiling point of -19°C, when sprayed, it volatilizes for the most part before adhering to the hair, has a poor adhesion to the hair though particles sprayed become finer, and dries up slowly, and therefore an effect of the styling resin cannot be sufficiently obtained.

The present invention has been made in view of such conventional problems, and it is an object of the present invention to provide a spray composition for hair styling and a spray product for hair styling both having an excellent drying property and an excellent setting power.

The spray composition for hair styling according to one embodiment of the present invention for solving the above-described problems is a spray composition for hair styling, comprising a styling resin and a solvent, the solvent comprising hydrofluoroolefin having a boiling point of 5 to 40°C and an alcohol.

Moreover, the spray product for hair styling according to one embodiment of the present invention for solving the above-described problems is a spray product for hair styling in which the above-described spray composition for hair styling and a compressed gas are filled in a pressure resistant container.

Furthermore, the spray product for hair styling according to one embodiment of the present invention for solving the above-described problems is a spray product for hair styling in which the above-described spray composition for hair styling is filled in a pressurized spray container.

### BRIEF DESCRIPTION OF THE DRAWINGS

FIG. 1 is a photograph of an appearance of a hair bundle after an adhesion test using a spray product for hair styling of Example 1.
FIG. 2 is a photograph of an appearance of the hair bundle after an adhesion test using a spray product for hair styling of Comparative example 1.

### EMBODIMENT FOR CARRYING OUT THE INVENTION

### <Spray composition for hair styling>

The spray composition for hair styling according to one embodiment of the present invention comprises a styling resin and a solvent. The solvent comprises hydrofluoroolefin having a boiling point of 5 to 40°C and an alcohol. Each of them will be described below.

### (Styling resin)

The styling resin is compounded for setting the hair.

The styling resin is not particularly limited. By way of an example, the styling resin includes anionic resin such as acrylic resin alkanolamine liquid such as (acrylate/diacetone acrylamide) copolymer AMP, (acrylate/diacetone acrylamide) copolymer TEA, (acrylate/alkyl acrylate/alkyl acrylamide) copolymer AMP, and (acrylate/alkyl acrylate/alkylacrylamide) copolymer TEA, amphoteric resin such as dialkylaminoethyl (meth)acrylate-(meth)acrylic acid ester copolymer and octylamide acrylate-hydroxypropyl acrylate-butyl methacrylate aminoethyl copolymer, cationic resin such as vinylpyrrolidone-N,N-dimethylaminoethyl methacrylic acid copolymer sulfate and hydroxyethylcellulose/dimethyldiallylammonium chloride, an emulsion type resin such as alkyl acrylate copolymer emulsion and alkyl acrylate-styrene copolymer emulsion, nonionic resin such as vinylpyrrolidone-vinyl acetate copolymer and hydroxyethyl acrylate-butyl acrylate-methoxyethyl acrylate copolymer, and the like. The styling resin may be used in combination.

A content (solid content) of the styling resin is not particularly limited. By way of an example, the content (solid content) of the styling resin is preferably 0.1% by mass or more, and more preferably 0.3% by mass or more, in the spray composition for hair styling. The content (solid content) of the styling resin is preferably 20% by mass or less, and more preferably 15% by mass or less, in the spray composition for hair styling. When the content (solid content) of the styling resin is within the above-described ranges, the spray composition for hair styling easily exhibits an excellent setting power.

### (Solvent)

The solvent is compounded for dissolving the styling resin in the spray composition for hair styling to form a uniform phase, forming particles having a size that allows for the spray composition for hair styling to easily adhere to the hair for spraying the composition uniformly, and making the composition to adhere to the hair to adjust drying property. Moreover, the solvent is compounded for adjusting an adhesion state, etc. of a film formed with the injected spray composition for hair styling.

The solvent comprises hydrofluoroolefin having a boiling point of 5 to 40°C and an alcohol.

### ·Hydrofluoroolefin having a boiling point of 5 to 40°C

Hydrofluoroolefin having a boiling point of 5 to 40°C dissolves a styling resin to form a stable spray composition for hair styling and is compounded for preventing clogging in a passage of a spray container, adjusting a particle size and drying property of a sprayed object, and the like.

Hydrofluoroolefin having a boiling point of 5 to 40°C is not particularly limited. By way of an example, hydrofluoroolefin having a boiling point of 5 to 40°C includes trans-1-chloro-3,3,3-trifluoropropene (HFO-1233zd(E), boiling point: 19°C), cis-1-chloro-3,3,3-trifluoropropene (HFO-1233zd(Z), boiling point: 39°C), cis-1-chloro-2,3,3,3-tetrafluoroolefin (HFO-1224yd(Z), boiling point: 15°C), and the like. Hydrofluoroolefin having a boiling point of 5 to 40°C may be used in combination.

A content of hydrofluoroolefin having a boiling point of 5 to 40°C is not particularly limited. By way of an example, the content of hydrofluoroolefin having a boiling point of 5 to 40°C is preferably 50% by mass or more, and more preferably 55% by mass or more, in the spray composition for hair styling. Moreover, the content of hydrofluoroolefin having a boiling point of 5 to 40°C is preferably 98% by mass or less, and more preferably 96% by mass or less, in the spray composition for hair styling. When the content of hydrofluoroolefin having a boiling point of 5 to 40°C is within the above-described ranges, the spray composition for hair styling enhances drying property of an adhered substance when adhering to the hair, the styling resin easily forms a film, and an excellent setting power is obtained. Furthermore, since the spray composition for hair styling has an excellent solubility for the styling resin, it is less likely to be clogged in a passage of a valve or a spray member. Here, according to common general knowledge, in order to improve drying property of the sprayed spray composition, it is considered that a liquefied gas having a low boiling point (for example, normal butane, isobutane, propane, a liquefied petroleum gas that is a mixture thereof, dimethyl ether, hydrofluoroolefin having a boiling point of less than 5°C, etc.) is compounded. However, in the present embodiment, contrary to such common technical knowledge, intentionally by compounding hydrofluoroolefin having an even higher boiling point of 5 to 40°C, instead of the conventionally used propellant having a boiling point of less than 5°C, it has been found that drying property of the spray composition sprayed onto the hair is improved. It has also been found that an excellent setting power can be thus obtained.

### · Alcohol

The alcohol stably dissolves the above-described styling resin and hydrofluoroolefin and is compounded as a solvent for forming a uniform spray composition for hair styling. Moreover, the alcohol is compounded for adjusting drying property, stretchability, etc. of a film formed with the sprayed spray composition for hair styling.

The alcohol is not particularly limited. By way of an example, the alcohol includes a monohydric alcohol having 2 to 3 carbon atoms such as ethanol and isopropanol.

A content of the alcohol is not particularly limited. By way of an example, the content of the alcohol is preferably 1% by mass or more, and more preferably 2% by mass or more, in the spray composition for hair styling. Moreover, the content of the alcohol is preferably 45% by mass or less, and more preferably 40% by mass or less, in the spray composition for hair styling. When the content of the alcohol is within the above-described ranges, the spray composition for hair styling is easily sprayed so as to have a particle size that is easy to adhere to the hair and is hard for a consumer to inhale. Furthermore, the sprayed particle easily obtains excellent drying property and setting power.

### ·Optional ingredients

The spray composition for hair styling of the present embodiment may comprise active ingredients as optional ingredients as appropriate, in addition to the above-described styling resin and solvent. The active ingredient is not particularly limited. By way of an example, the active ingredient includes UV absorbers such as hexyl diethylaminohydroxybenzoylbenzoate, 2-ethylhexyl paramethoxycinnamate, ethylhexyl triazone, oxybenzone, hydroxybenzophenone sulfonic acid, sodium dihydroxybenzophenone sulfonate, and dihydroxybenzophenone; UV scattering agents such as zinc oxide, titanium oxide, and octyltrimethoxysilane-coated titanium oxide; vitamins such as retinol, retinol acetate, retinol palmitate, calcium pantothenate, ascorbic acid, sodium ascorbate, dl-α-tocopherol, tocopherol acetate, tocopherol, tocopherol nicotinate, dibenzoylthiamine, riboflavin, and mixtures thereof; various extracts such as a Swertia japonica extract and a rosemary extract; moisturizing agents such as glycerin, 1,3-butylene glycol, propylene glycol, hexylene glycol, dipropylene glycol, diglycerin, and hyaluronic acid; flavoring agents such as floral, green, citrus green, green floral, citrus, rose, rosewood, herbal wood, lemon, and peppermint; pigments such as aluminum powder, silica, and iron oxide; dyes such as Yellow No. 4, Yellow No. 5, Yellow No. 202, Yellow No. 203, Red No. 2, Red No. 3, Blue No. 1, and Blue No. 2; surfactants such as polysorbate, polyglycerol fatty acid ester, and polyoxyethylene alkyl ether; chelating agents such as disodium edetate; preservatives such as paraben and phenoxyethanol; various oil components such as oils and fats, an ester oil, a higher fatty acid, a higher alcohol, hydrocarbon, and silicone, and the like.

When an active ingredient is compounded, a content of the active ingredient is not particularly limited. By way of an example, the content of the active ingredient is preferably 0.1% by mass or more, and more preferably 0.3% by mass or more, in the spray composition for hair styling. Moreover, the content of the active ingredient is preferably 20% by mass or less, and more preferably 15% by mass or less, in the spray composition for hair styling. When the content of the active ingredient is within the above-described ranges, the resulting spray composition for hair styling easily obtains a desired effect due to the compounding of the active ingredient.

Referring back to the description of the spray composition for hair styling as a whole, a mass ratio of hydrofluoroolefin having a boiling point of 5 to 40°C to the alcohol is preferably 50:50 to 99:1, and more preferably 55:45 to 98:2. When the mass ratio of hydrofluoroolefin having a boiling point of 5 to 40°C and the alcohol is within the above-described ranges, the spray composition for hair styling is particularly excellent in drying property and can easily obtain a more excellent setting power.

A method of preparing a spray composition for hair styling is not particularly limited. The spray composition for hair styling can be prepared by a conventionally known method. For example, the spray composition for hair styling can be prepared by mixing optional ingredients such as the above-described styling resin, alcohol, and active ingredient, and adding hydrofluoroolefin to the mixture.

As described above, the spray composition for hair styling of the present embodiment easily forms a particle of a size that easily adheres to the hair, in which hydrofluoroolefin easily remains in the adhered substance adhered to the hair. The adhered substance is thereby dried in a very short time. Moreover, when the adhered substance is dried, a film of the styling resin is easily formed on the hair, easily obtaining an excellent setting power.

### <Spray product for hair styling>

The above-described spray composition for hair styling can be used as a spray product for hair styling by being filled in an appropriate container or the like.

### (First embodiment)

The spray product for hair styling according to one embodiment of the present invention is a spray product for hair styling in which the above-described spray composition for hair styling and a compressed gas are filled in a pressure resistant container.

### ·Compressed gas

A compressed gas is compounded as a propellant for pressurizing and spraying the spray composition for hair styling to the outside.

The compressed gas is not particularly limited. By way of example, the compressed gas includes, nitrogen, air, oxygen, hydrogen, carbon dioxide, nitrous oxide, and the like. In particular, when using carbon dioxide or nitrous oxide which dissolves in hydrofluoroolefin having a boiling point of 5 to 40°C in a large amount, a vapor tap hole can be provided in a housing which will be described later. As a result, the spray product for hair styling easily forms a particle of a size that easily adheres to the hair and can shorten or even eliminate a flame length in a flame length test.

The compressed gas is filled so that a pressure in the container at 25°C is preferably 0.3 MPa or more, and more preferably 0.4 MPa or more. Moreover, the compressed gas is filled so that the pressure in the container at 25°C is preferably 1.0 MPa or less, and more preferably 0.8 MPa or less. When the compressed gas is filled so that the pressure is within the above-described ranges, the spray composition for hair styling easily forms a particle of a size that easily adheres to the hair and is easily sprayed in an appropriate amount.

The spray composition for hair styling may be used in combination with a liquefied gas as long as a drying effect of hydrofluoroolefin having a boiling point of 5 to 40°C on the adhered substance is not impaired.

The liquefied gas is not particularly limited. By way of an example, the liquefied gas includes normal butane, isobutane, propane, a liquefied petroleum gas that is a mixture thereof, dimethyl ether, hydrofluoroolefin having a boiling point of less than 5°C (HFO-1234ze (E), boiling point: -19°C, or HFO-1234yf, boiling point: -30°C), and the like. The liquefied gas may be used in combination. A mass ratio of the spray composition for hair styling to the liquefied gas (spray composition for hair styling/liquefied gas) is preferably 95:5 to 80:20. When the mass ratio of the liquefied gas increases, in the spray composition for hair styling, evaporation of hydrofluoroolefin having a boiling point of 5 to 40°C is facilitated, and drying property of the adhered substance adhered to the hair becomes easily decreased.

A method of producing the spray product for hair styling of this embodiment is not particularly limited. By way of an example, the spray product for hair styling can be prepared by filling a pressure resistant container with a spray composition for hair styling, attaching a valve to an opening of the pressure resistant container, and filling with a compressed gas from the valve. Moreover, the spray product for hair styling may be produced by attaching an injection member to a stem of the valve.

The pressure resistant container is a container filled with a spray composition for hair styling and has a bottomed cylindrical shape. A valve is attached to the opening of the pressure resistant container.

A material of the pressure resistant container is not particularly limited. By way of an example, the material of the pressure resistant container includes metal such as aluminum and tin, various synthetic resins, a pressure resistant glass, and the like.

The valve is a member for closing and sealing the opening of the pressure resistant container. Moreover, the valve mainly comprises a housing held by a mounting cup mounted to the opening of the pressure resistant container, a stem having a stem hole communicating between the inside and outside of the pressure resistant container formed, and a stem rubber for closing the stem hole and which is attached around the stem hole. The housing houses the stem, the stem rubber, and a spring that biases the stem upward. An injection member for injecting the spray composition for hair styling is attached to the upper end of the stem.

The housing has a substantially cylindrical shape and is provided at its lateral side with a gas phase introduction hole (vapor tap hole) for introducing a gas phase of the spray composition and at its lower part with a liquid phase introduction hole for introducing a liquid phase of the spray composition.

A dimension (cross-sectional area) of the gas phase introduction hole is not particularly limited. By way of an example, the dimension (cross-sectional area) of the gas phase introduction hole is preferably 0.01 mm² or more, and more preferably 0.03 mm² or more. Moreover, the dimension (cross-sectional area) of the gas phase introduction hole is preferably 0.20 mm² or less, and more preferably 0.15 mm² or less. When the dimension (cross-sectional area) of the gas phase introduction hole is within the above-described ranges, a compressed gas is introduced into the housing at the time of injection, making it easier to form a particle of a size that easily adheres to the hair, and in the flame length test which measures an extension of flame when spraying toward the flame, the flame length can be shortened or even eliminated, allowing for increase in safety of the injected spray composition.

A dimension (cross-sectional area) of the liquid phase introduction hole is not particularly limited. By way of an example, the dimension (cross-sectional area) of the liquid phase introduction hole is preferably 0.1 mm² or more, and more preferably 0.2 mm² or more. Moreover, the dimension (cross-sectional area) of the liquid phase introduction hole is preferably 3.5 mm² or less, and more preferably 3.0 mm² or less. When the dimension (cross-sectional area) of the liquid phase introduction hole is within the above-described ranges, a liquid phase portion of the sprayed composition is easily introduced into the housing in an appropriate amount, stabilizing the injection state.

Besides, according to common general knowledge, in a case of an aerosol product containing a compressed gas, most of the compressed gas is in the gas phase, so that the compressed gas is discharged from the gas phase introduction hole along with the injection to reduce the pressure, by which the injection cannot be completed to the end. On the other hand, when the spray composition of this embodiment contains a carbon dioxide gas or a nitrous oxide gas as a compressed gas, an amount of the gas dissolved in a solvent is as large as 0.01 g/mL or more, and preferably 0.02 g/mL or more, and therefore, even if the compressed gas in the gas phase is discharged from the gas phase introduction hole at the time of injection, the compressed gas dissolved in the solvent volatilizes to increase the pressure, by which the injection can be completed to the end.

In this case, the compressed gas introduced into the housing through the gas phase introduction hole at the time of injection and the liquid phase portion of the spray composition introduced into the housing through the liquid phase introduction hole are mixed in the housing. The spray composition is sprayed with a large amount of compressed gas dissolved in a solvent comprising hydrofluoroolefin having a boiling point of 5 to 40°C and an alcohol being mixed with a gaseous compressed gas, so that a particle of a size that easily adheres to the flame is easily formed, and in the flame length test which measures an extension of flame when spraying toward the flame, the flame length can be shortened or even eliminated, allowing for increase in safety of the injected spray composition.

The injection member (actuator) is a member for injecting the spray composition for hair styling by operating opening and closing of a valve and is attached to the upper end of the stem. The injection member mainly comprises a nozzle part in which an injection hole is formed and an operation part operated by user's fingers or the like. The spray composition for hair styling is injected from the injection hole. The number and shape of the injection hole are not particularly limited. A plurality of injection holes may be provided. Moreover, the shape of the injection hole may be substantially circular, substantially rectangular, or the like.

In the spray product for hair styling of this embodiment, when the injection member is pushed down, the stem of the valve is pushed downward. The stem rubber thereby bends downward to open the stem hole. As a result, the inside and outside of the pressure resistant container communicate with each other. When the inside and outside of the pressure resistant container communicate with each other, the spray composition for hair styling is taken into the housing by a pressure difference between pressures inside and outside the pressure resistant container, and then passes through the stem hole and the passage in the stem, and is sent to the injection member, followed by injected from the injection hole.

As such, the spray product for hair styling can spray the spray composition for hair styling under a pressure of a compressed gas. The sprayed particle is easily sprayed which has a size that easily adheres to the hair and is hard for a consumer to inhale. Moreover, the spray composition for hair styling comprises a styling resin and a solvent comprising hydrofluoroolefin having a boiling point of 5 to 40°C and an alcohol. The spray composition for hair styling easily adheres to the hair. In addition, the adhered substance is excellent in drying property, and the styling resin easily forms a film on the hair, obtaining an excellent setting power.

### (Second embodiment)

The spray product for hair styling according to one embodiment of the present invention is a spray product for hair styling, in which the above-described spray composition for hair styling is filled in a container body (an example of a pressurized spray container).

A method of producing the spray product for hair styling of this embodiment is not particularly limited. By way of an example, the spray product for hair styling can be prepared by filling the container body with a spray composition for hair styling and attaching a pressurized valve to the opening of the container body. Moreover, the spray product for hair styling may be produced by attaching an injection member to a stem of the pressurized valve.

As the container body, the similar one as the pressure resistant container described above may be used. Moreover, since the container body does not need to adopt such a container having a high pressure resistant that can withstand a higher internal pressure, the weight of the container can be reduced, and amounts of materials used can be reduced. A screw can be provided in the opening of the container body, and the pressurized valve can be attached by screwing.

The pressurized valve is a member for closing the opening of the container body, taking in the spray composition for hair styling in the container body, and pressurizing and sending it to the injection member for spraying. The pressurized valve comprises a housing for storing the spray composition for hair styling to be sprayed, a pressure member for pressurizing the spray composition for hair styling in the housing, and a cap for housing the pressure member in the housing and mounted to the container body. In order to prevent volatilization of hydrofluoroolefin having a boiling point of 5 to 40°C, it is preferable that the housing is configured to have no air introduction holes formed or have a packing provided around an air introduction hole to block a flow from inside the container body to inside the housing. The pressure member comprises a stem, a piston member, a spring that biases the stem upward, and a ball valve. An injection member is attached to the stem.

When the injection member is pushed down, the stem and the piston member are lowered. Since the introduction hole in the lower part of the housing is closed with the ball valve, the spray composition for hair styling in the housing is pressurized with the stem and the piston member. When the lowering movement of the piston member is stopped with the pressurized spray composition for hair styling, a gap is formed between the stem and the piston member. The spray composition for hair styling is sent to the injection member through this gap and then injected from the injection hole.

As such, the spray product for hair styling can pressurize and spray the spray composition for hair styling. Moreover, the spray composition for hair styling comprises a styling resin and a solvent comprising hydrofluoroolefin having a boiling point of 5 to 40°C and an alcohol. The spray composition for hair styling easily adheres to the hair. In addition, the adhered substance is excellent in drying property, and the styling resin easily forms a film on the hair, obtaining an excellent setting power.

The spray composition for hair styling of this embodiment is preferably sprayed in a mist form which has a particle size that easily adheres to the hair. An average particle size of the spray composition is preferably 5 to 50 µm, and more preferably 6 to 45 µm. When the average particle size is less than 5 µm, the spray composition is easily inhaled, and when it exceeds 50 µm, drying property tends to be deteriorated.

The embodiments of the present invention have been described above. The present invention is not particularly limited to the above-described embodiments. Besides, the above-described embodiments mainly describe an invention having the following configuration.
(1) A spray composition for hair styling, comprising a styling resin and a solvent, wherein the solvent comprises hydrofluoroolefin having a boiling point of 5 to 40°C and an alcohol.
   According to such a configuration, the spray composition for hair styling easily forms a particle of a size that easily adheres to the hair, in which hydrofluoroolefin easily remain in the adhered substance adhered to the hair. As a result, the adhered substance is dried in a very short time, the styling resin easily forms a film on the hair and easily exhibits its effect, easily obtaining an excellent setting power. In addition, since the solvent has an excellent solubility for the styling resin, the styling resin is less likely to be clogged in the passage of the valve or the spray member.
(2) The spray composition for hair styling of (1), wherein a content of the hydrofluoroolefin is 50 to 98% by mass in the spray composition for hair styling.
   According to such a configuration, the spray composition for hair styling easily obtains more excellent drying property and setting power.
(3) The spray composition for hair styling of (1) or (2), wherein a mass ratio of the hydrofluoroolefin to the alcohol is from 50:50 to 99:1.
   According to such a configuration, the spray composition for hair styling is particularly excellent in drying property and easily obtains a more excellent setting power.
(4) A spray product for hair styling, wherein the spray composition for hair styling of any one of (1) to (3) and a compressed gas are filled in a pressure resistant container.
   According to such a configuration, the spray product for hair styling can continuously spray the spray composition for hair styling under a pressure of the compressed gas. The sprayed particle is easily sprayed which has a size that easily adheres to the hair and is hard for a consumer to inhale. Moreover, the spray composition for hair styling comprises a styling resin and a solvent comprising hydrofluoroolefin having a boiling point of 5 to 40°C and an alcohol. The spray composition for hair styling easily adheres to the hair. In addition, the adhered substance is excellent in drying property, and the styling resin easily forms a film on the hair, obtaining an excellent setting power.
(5) The spray product for hair styling of (4), wherein the pressure resistant container comprises a valve provided with a gas phase introduction hole for introducing a gas phase of the spray composition.
   According to such a configuration, the compressed gas introduced into the housing through the gas phase introduction hole at the time of injection and the liquid phase portion of the spray composition introduced into the housing through the liquid phase introduction hole are mixed in the housing. The spray product is sprayed with a large amount of compressed gas dissolved in a solvent comprising hydrofluoroolefin having a boiling point of 5 to 40°C and an alcohol being mixed with a gaseous compressed gas, so that a particle of a size that easily adheres to the flame is easily formed, and in the flame length test which measures an extension of flame when spraying toward the flame, the flame length can be shortened or even eliminated, allowing for increase in safety of the injected spray composition.
(6) A spray product for hair styling, wherein the spray composition for hair styling of any one of (1) to (3) is filled in a pressurized spray container.
   According to such a configuration, the spray product for hair styling can pressurize and spray the spray composition for hair styling. The sprayed particle is easily sprayed which has a size that easily adheres to the hair and is hard for a consumer to inhale. Moreover, the spray composition for hair styling comprises a styling resin and a solvent comprising hydrofluoroolefin having a boiling point of 5 to 40°C and an alcohol. The spray composition for hair styling easily adheres to the hair. In addition, the adhered substance is excellent in drying property, and the styling resin easily forms a film on the hair, obtaining an excellent setting power.

### EXAMPLE

The present invention will be described more specifically with reference to examples below. The present invention is not limited to these examples at all.

### (Example 1)

A 100 g of concentrate 1 was prepared and filled in an aluminum pressure resistant container, according to formulations described in Table 1 below (unit: % by mass). Next, a valve (stem hole: ϕ0.4, housing lower hole: ϕ0.6, vapor tap hole: ϕ0.3) was fixed to the opening of the pressure resistant container to seal it. Furthermore, a carbon dioxide gas was filled from the stem, and a spray member (spray hole: ϕ0.43, with a mechanical breakup mechanism) was attached to the stem to prepare a spray product for hair styling. A pressure inside the container was 0.5 MPa (25°C). Besides, an amount of the carbon dioxide gas dissolved in the solvent was 0.040 g/mL.

**Table 1**

| Concentrate name | Concentrate | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| (Acrylate/diacetone acrylamide) copolymer AMP/ethanol (*1) | 6.0 | 9.0 | 12.0 | 3.0 | 7.5 | 10.0 | 30.0 | 6.0 | 14.1 | 4.8 | 3.0 | 4.0 |
| Ethanol | 14.0 | 21.0 | 28.0 | 7.0 | 12.5 | 10.0 | 70.0 | 94.0 | 32.8 | 3.2 | 2.0 | 4.0 |
| Hydrofluoroolefin (*2) | 80.0 | 70.0 | 60.0 | 90.0 | 80.0 | 80.0 | - | - | - | 92.0 | 95.0 | 92.0 |
| Isopentane (*3) | - | - | - | - | - | - | - | - | 53.1 | - | - | - |
| Total | 100.0 | 100.0 | 100.0 | 100.0 | 100.0 | 100.0 | 100.0 | 100.0 | 100.0 | 100.0 | 100.0 | 100.0 |

### (Examples 2 to 4)

Spray products for hair styling were prepared in the similar manner as in Example 1, except that formulations of concentrates were changed to the formulations described in Table 1 (concentrates 2 to 4). Besides, amounts of carbon dioxide dissolved in the solvent were 0.034 g/mL in Example 2, 0.032 g/mL in Example 3, and 0.042 g/mL in Example 4.

### (Example 5)

A 100 g of concentrate 1 described in Table 1 was filled in an aluminum pressure resistant container. Next, a valve (stem hole: ϕ0.4, housing lower hole: ϕ2.0, vapor tap hole: none) was fixed to the opening of the pressure resistant container to seal it. Furthermore, nitrogen was filled from the stem, and a spray member (spray hole: ϕ0.43, with a mechanical breakup mechanism) was attached to the stem to prepare a spray product for hair styling. A pressure inside the container was 0.5 MPa (25°C). Besides, an amount of nitrogen dissolved in the solvent was 0.001 g/mL.

### (Example 6)

A 100 g of concentrate 1 described in Table 1 was filled in a synthetic resin container and filled with nitrogen, a pressurized valve (with specification in which the air introduction hole of the housing was closed) was attached to the opening of the synthetic resin container by screwing and sealed, and a spray member (spray hole: ϕ0.45, with a mechanical breakup mechanism) was attached to the stem, to prepare a spray product for hair styling. A pressure inside the container was 0.5 MPa (25°C). Besides, an amount of nitrogen dissolved in the solvent was 0.001 g/mL.

### (Examples 7 to 8)

Spray products for hair styling were prepared in the similar manner as in Example 1, except that formulations of concentrates were changed to the formulations described in Table 1 (concentrates 5 to 6). Besides, amounts of carbon dioxide gas dissolved in the solvent were 0.039 g/mL in Example 7 and 0.038 g/mL in Example 8.

### (Example 9)

A 100 g of concentrate 10 described in Table 1 was filled in an aluminum pressure resistant container. Next, a valve (stem hole: ϕ0.4, housing lower hole: ϕ0.6, vapor tap hole: ϕ0.3) was fixed to the opening of the pressure resistant container to seal it. Furthermore, a carbon dioxide gas was filled from the stem, and a spray member (spray hole: ϕ0.43, with a mechanical breakup mechanism) was attached to the stem to prepare a spray product for hair styling. A pressure inside the container was 0.45 MPa (25°C). Besides, an amount of the carbon dioxide gas dissolved in the solvent was 0.039 g/mL.

### (Example 10)

A spray product for hair styling was prepared in the similar manner as in Example 9, except that concentrate 11 described in Table 1 was used. Besides, an amount of the carbon dioxide gas dissolved in the solvent was 0.039 g/mL.

### (Example 11)

A 100 g of concentrate 12 described in Table 1 was filled in an aluminum pressure resistant container. Next, a valve (stem hole: ϕ0.4, housing lower hole: ϕ1.55, vapor tap hole: ϕ0.4) was fixed to the opening of the pressure resistant container to seal it. Furthermore, a carbon dioxide gas was filled from the stem, and a spray member (spray hole: ϕ0.43, with a mechanical breakup mechanism) was attached to the stem to prepare a spray product for hair styling. A pressure inside the container was 0.45 MPa (25°C). Besides, an amount of the carbon dioxide gas dissolved in the solvent was 0.039 g/mL.

### (Example 12)

A 100 g of concentrate 12 described in Table 1 was filled in an aluminum pressure resistant container. Next, a valve (stem hole: ϕ0.4, housing lower hole: ϕ2.0, vapor tap hole: none) was fixed to the opening of the pressure resistant container to seal it. Furthermore, nitrogen was filled from the stem, and a spray member (spray hole: ϕ0.43, with a mechanical breakup mechanism) was attached to the stem to prepare a spray product for hair styling. A pressure inside the container was 0.45 MPa (25°C). Besides, an amount of nitrogen dissolved in the solvent was 0.001 g/mL.

### (Comparative example 1)

A 20 g of concentrate 7 described in Table 1 was filled in an aluminum pressure resistant container. Next, a valve (stem hole: ϕ0.4, housing lower hole: ϕ0.6, vapor tap hole: ϕ0.3) was fixed to the opening of the pressure resistant container to seal it. Furthermore, a 80 g of HFO-1234ze (E) was filled from the stem, and a spray member (spray hole: ϕ0.43, with a mechanical breakup mechanism) was attached to the stem to prepare a spray product for hair styling.

### (Comparative example 2)

A 50 g of concentrate 7 described in Table 1 was filled in an aluminum pressure resistant container. Next, a valve (stem hole: ϕ0.4, housing lower hole: ϕ0.6, vapor tap hole: ϕ0.3) was fixed to the opening of the pressure resistant container to seal it. Furthermore, a 50 g of dimethyl ether (DME) was filled from the stem, and a spray member (spray hole: ϕ0.43, with a mechanical breakup mechanism) was attached to the stem to prepare a spray product for hair styling.

### (Comparative example 3)

A 100 g of concentrate 8 described in Table 1 was filled in a synthetic resin container and filled with nitrogen, and a valve (stem hole: ϕ0.4, housing lower hole: ϕ2.0, vapor tap hole: none) was fixed to the opening of the pressure resistant container to seal it. Furthermore, nitrogen was filled from the stem, and a spray member (spray hole: ϕ0.43, with a mechanical breakup mechanism) was attached to the stem to prepare a spray product for hair styling. A pressure inside the container was 0.5 MPa (25°C).

### (Comparative example 4)

A 100 g of concentrate 8 described in Table 1 was filled in a synthetic resin container and filled with nitrogen, a pressurized valve (with specification in which the air introduction hole of the housing was closed) was attached to the opening of the synthetic resin container by screwing and sealed, and a spray member (spray hole: ϕ0.45, with a mechanical breakup mechanism) was attached to the stem, to prepare a spray product for hair styling. A pressure inside the container was 0.5 MPa (25°C).

### (Comparative example 5)

A 100 g of concentrate 9 described in Table 1 was filled in a synthetic resin container and filled with a carbon dioxide gas, and a valve (stem hole: ϕ0.4, housing lower hole: ϕ0.6, vapor tap hole: ϕ0.3) was fixed to the opening of the pressure resistant container to seal it. Furthermore, a carbon dioxide gas was filled from the stem, and a spray member (spray hole: ϕ0.43, with a mechanical breakup mechanism) was attached to the stem to prepare a spray product for hair styling. A pressure inside the container was 0.5 MPa (25°C).

### (Comparative example 6)

A 100 g of concentrate 9 described in Table 1 was filled in a synthetic resin container and filled with nitrogen, and a valve (stem hole: ϕ0.4, housing lower hole: ϕ2.0, vapor tap hole: none) was fixed to the opening of the pressure resistant container to seal it. Furthermore, nitrogen was filled from the stem, and a spray member (spray hole: ϕ0.43, with a mechanical breakup mechanism) was attached to the stem to prepare a spray product for hair styling. A pressure inside the container was 0.5 MPa (25°C).

The spray products for hair styling prepared in Examples 1 to 12 and Comparative examples 1 to 6 were used to measure adhesion, permeability, average particle size, drying property, condition of adhered substance, setting power, clogging, and flame length by the following evaluation methods. Table 2 shows the results.

### <Adhesion>

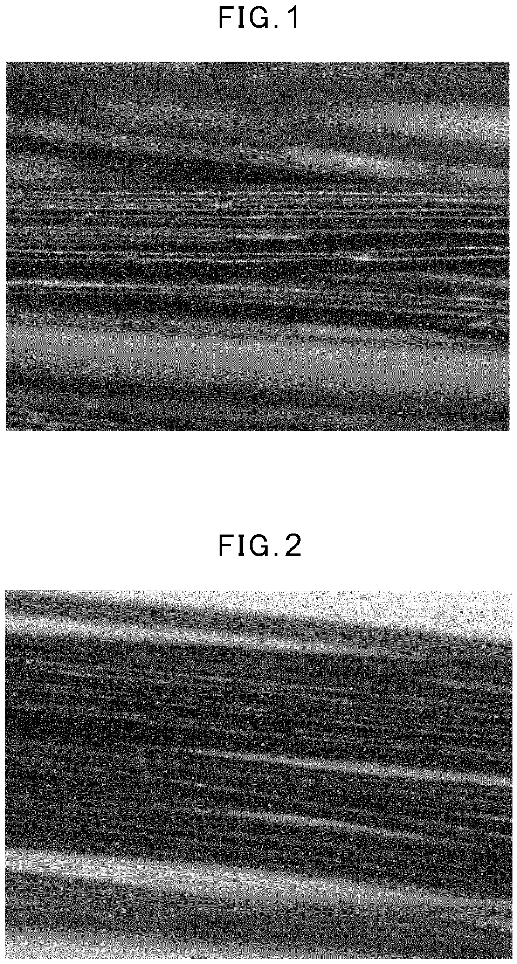

The spray product for hair styling was adjusted to 25°C and sprayed onto a hair bundle from a distance of 10 cm. Adhesion to the hair bundle was evaluated according to the following evaluation criteria. Besides, FIG. 1 is a photograph of an appearance of a hair bundle after an adhesion test using a spray product for hair styling of Example 1. FIG. 2 is a photograph of an appearance of the hair bundle after an adhesion test using a spray product for hair styling of Comparative example 1.

### (Evaluation criteria)

o: The spray composition for hair styling adhered uniformly to the entire hair bundle.
△: The spray composition for hair styling adhered to a portion of the hair bundle in a granular form and did not uniformly adhere to the entire hair bundle.
×: The spray composition for hair styling sparsely adhered to a portion of the hair bundle in a granular form and did not uniformly adhere to the entire hair bundle.

### <Permeability>

The spray product for hair styling was adjusted to 25°C and sprayed onto a hair bundle from a distance of 10 cm. Permeability to the back side of the hair bundle was evaluated according to the following evaluation criteria.

### (Evaluation criteria)

o: The spray composition for hair styling sprayed onto the hair bundle permeated to the back side.
×: The spray composition for hair styling sprayed onto the hair bundle did not permeate to the back side.

### <Average particle size>

The spray product for hair styling was adjusted to 25°C, and an average particle size of sprayed particles at a distance of 15 cm from the injection hole was measured using a particle size distribution measuring device (LDSA-3400A) manufactured by MicrotracBEL Corp.

### <Drying property>

The spray product for hair styling was adjusted to 25°C and sprayed onto the hair bundle from a distance of 10 cm so as to cover the entire hair bundle in one reciprocal motion. Drying property of the hair bundle was evaluated according to the following evaluation criteria.

### (Evaluation criteria)

⊚: The hair bundle dried within 5 minutes.
∘: The hair bundle dried in 5 to 10 minutes.
×: The hair bundle did not dry even after 10 minutes.

### <Condition of adhered substance>

The spray composition for hair styling was adjusted to 25°C and sprayed onto the hair. Condition of the adhered substance was evaluated according to the following evaluation criteria.

### (Evaluation criteria)

∘: No resin was deposited even after drying.
×: Resin was deposited after drying.

### <Setting power>

The spray product for hair styling was adjusted to 25°C and sprayed from a distance of 10 cm with the hair bundle raised vertically from a mannequin. The hair bundle was lifted until it dried, and after drying, the hand was released, and an angle of the hair bundle was confirmed to evaluate setting power according to the following evaluation criteria. Besides, the angle of the hair bundle refers to an angle between a hair bundle and a horizontal plane with a vertical direction being as 90°.

### (Evaluation criteria)

⊚: The angle of the hair bundle was 90 to 60°.
∘: The angle of the hair bundle was 59 to 30°.
×: The angle of the hair bundle was 29 to 0°.

### <Clogging>

The spray product for hair styling stored at 25°C was sprayed for 1 second twice a day for one consecutive month, and clogging in a nozzle hole was confirmed and evaluated according to the following evaluation criteria.

### (Evaluation criteria)

∘: There was no clogging in the nozzle hole, allowing for the spray composition for hair styling to be stably sprayed.
×: There was clogging in the nozzle hole, not allowing for the spray composition for hair styling to be stably sprayed.

### <Flame length>

The spray product for hair styling was adjusted to 25°C and sprayed from 15 cm behind to a flame of 5 cm in height to evaluate a flame extension (flame length) according to the following evaluation criteria.

### (Evaluation criteria)

⊚: No flame length was observed.
∘: The flame length was less than 25 cm.
△: The flame length was 25 cm or more and less than 45 cm.
×: The flame length was 45 cm or more.

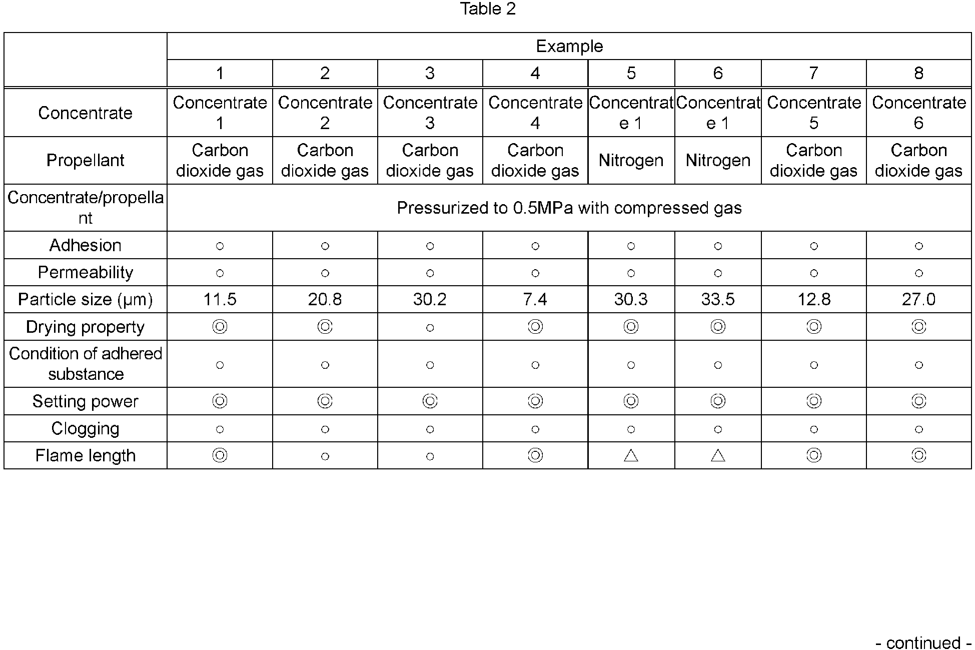

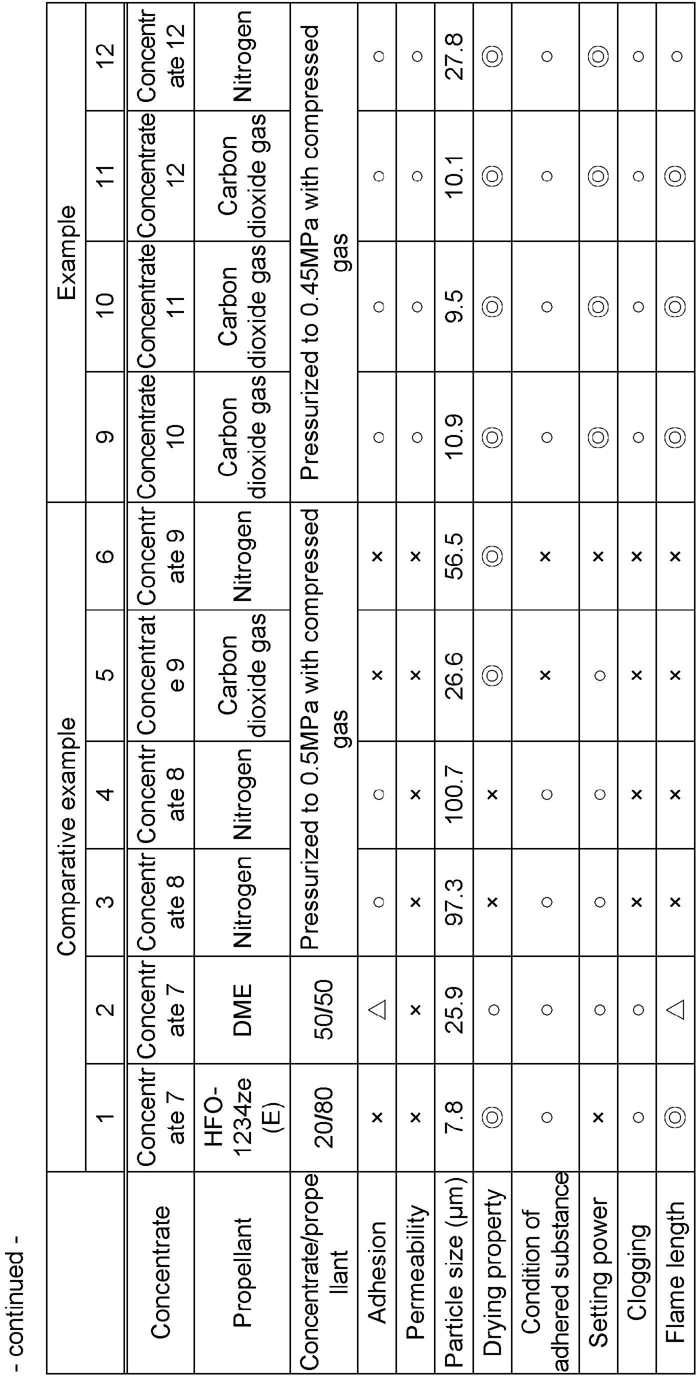

As shown in Table 2, the spray compositions for hair styling of Examples 1, 2, 4 to 8 did not deposit resin even after drying, had an excellent adhesion, had good drying property and permeability to a hair bundle, and exhibited an excellent setting power. The spray composition for hair styling of Example 3 did not deposit resin even after drying, had an equivalent drying property to that of the conventional hair spray (Comparative example 2), had an excellent adhesion, and exhibited an excellent setting power with a particle size that is hardly inhaled. On the other hand, the spray composition for hair styling of Comparative example 1, which used hydrofluoroolefin (HFO-1234ze(E)) having a boiling point of -19°C instead of hydrofluoroolefin having a boiling point of 5 to 40°C, had a poor adhesion, and had poor permeability to the hair bundle and setting power. The spray composition for hair styling of Comparative example 2, which is a common hair spray, was not as good in adhesion compared with Examples, and also had a poor permeability to the hair bundle. The spray compositions for hair styling of Comparative examples 3 and 4, in which hydrofluoroolefin having a boiling point of 5 to 40°C was not compounded, had an equivalent setting power to that of Comparative example 2, had poor drying property and permeability to the hair bundle, and caused clogging, which made the spray compositions for hair styling not to be sprayed to the end. The spray compositions for hair styling of Comparative examples 5 and 6, which used isopentane instead of hydrofluoroolefin having a boiling point of 5 to 40°C, had poor adhesion and permeability to the hair bundle, and caused clogging, which made the spray compositions for hair styling not to be sprayed to the end. In addition, according to the photograph of the appearance of the hair bundle after the adhesion test, as shown in FIG. 1, it was found that, as a result of the adhesion test in which the spray composition for hair styling of Example 1 was used, the spray composition for hair styling uniformly adhered to the entire hair bundle, forming a film. On the other hand, as shown in FIG. 2, it was found that, as a result of the adhesion test in which the spray composition for hair styling of Comparative example 1 was used, the spray composition for hair styling did not uniformly adhere to the entire hair bundle, not sufficiently forming a film. The spray compositions for hair styling of Examples 9 to 12 did not deposit resin even after drying, had an excellent adhesion, had good drying property and permeability to a hair bundle, and exhibited an excellent setting power. In addition, in the spray compositions for hair styling of Examples 1, 4, 7, 8, 9 to 11, no flame length was observed in flame length tests. In particular, the spray compositions for hair styling of Examples 9 to 11 had deflagration density of 300 g/m³ or more and a very high safety against fire in an enclosed space ignition test specified in United Nations Recommendations on the Transport of Dangerous Goods, Manual of Tests and Criteria, Paragraph 31.5.

## Claims

1. A spray composition for hair styling, comprising a styling resin and a solvent,
wherein the solvent comprises hydrofluoroolefin having a boiling point of 5 to 40°C and an alcohol.

2. The spray composition for hair styling of claim 1, wherein a content of the hydrofluoroolefin is 50 to 98% by mass in the spray composition for hair styling.

3. The spray composition for hair styling of claim 1 or 2, wherein a mass ratio of the hydrofluoroolefin to the alcohol is from 50:50 to 99:1.

4. A spray product for hair styling, wherein the spray composition for hair styling of any one of claims 1 to 3 and a compressed gas are filled in a pressure resistant container.

5. The spray product for hair styling of claim 4, wherein the pressure resistant container comprises a valve provided with a gas phase introduction hole for introducing a gas phase of the spray composition.

6. A spray product for hair styling, wherein the spray composition for hair styling of any one of claims 1 to 3 is filled in a pressurized spray container.
